# EUROPEAN PATENT APPLICATION

(11) **EP 0 611 827 A1**
(43) Date of publication of application: **24.08.1994**
(21) Application number: 94101911.9
(22) Date of filing: 08.02.1994
(51) Int. Cl.: C12Q 1/00, G01N 27/30

(54) **Substrate sensitive membrane having thin metal film layer and enzyme electrode using the same**

(30) Priority: 13.02.1993 JP 64608/93
(71) Applicant: NIPPON SUISAN KAISHA, LTD., Tokyo (JP)
(72) Inventor: Ohashi, Eiji, Tokyo (JP); Karube, Isao, Kawasaki-shi, Kanagawa (JP); Tamiya, Eiichi, Setagaya-ku, Tokyo (JP)
(74) Representative: Hansen, Bernd, Dr. Dipl.-Chem.

(57) **Abstract**

An enzyme-immobilized membrane, namely a substrate sensitive membrane, on which a thin metal film layer is directly formed is disclosed. The membrane may contain two or more of immobilized enzymes. It can be applied to an enzyme electrode which is excellent in uniformity, miniaturization and safety.

## Description

### FIELD OF THE INVENTION

This invention relates to a substrate sensitive membrane having a thin metal film layer, specifically a substrate sensitive chitin membrane having a thin metal film layer, more specifically a substrate sensitive β-chitin membrane having a thin metal film layer, and to an enzyme electrode using the membrane.

### BACKGROUND OF THE INVENTION

Enzyme sensor is a measuring device which is constructed in principle by equipping an electrode with an immobilized enzyme membrane, namely a substrate sensitive membrane, and its basic function is to detect a compound formed or digested by an enzyme reaction using an electro-chemical device and convert the detected data into electronic signals, thereby indirectly measuring a substrate specific for the enzyme. In this instance, the enzyme membrane functions as a molecular recognition site, and the electrochemical device functions as a transducer.

Since the enzyme sensor makes use of excellent substrate specificity of an enzyme employed, it has excellent selectivity of substances to be measured. Various enzymes which are available have been applied to enzyme sensors for measuring the corresponding substrates.

Though the enzyme sensor has originally been developed with the aim of measuring a single substrate of interest, great concern has recently been directed toward the development of a multi-functional enzyme sensor which can measure a plurality of components simultaneously.

Studies of the enzyme electrode, on the other hand, are now directed toward the prolongation of the shelf life and increase in the number of substances to be analyzed. While one of the characteristic advantages of the enzyme electrode is its economical cost for analysis, it has been desired to develop techniques for prolonging the shelf life and simplifying analytical operation.

Since demands for the enzyme electrode seem to increase more and more in the future especially in the field of clinical inspections, such as emergency clinical test and bedside inspection, it is necessary to develop various types of electrodes. It has become more and more important to develop enzyme electrodes comprising a single support membrane having a plurality of enzymes immobilized thereon.

In addition, enzyme membranes are most important for the development of an enzyme sensor, because properties of the enzyme sensor, such as the rate of response, sensitivity and stability, vary greatly depending on the characteristics of the enzyme membrane to be used. Especially, development of carriers for immobilization for sensor use is a current subject to be accomplished.

When an enzyme is immobilized on a carrier in an industrial scale, it is required that the immobilization operation can be carried out simply and easily, the immobilized enzyme can be maintained stably for a long time and the carrier to be used is inexpensive. Especially, the carrier must have high substrate permeability and be moldable into a thin membrane for an enzyme electrode use.

However, the prior art carriers have disadvantages in that cross linking is necessary for the immobilization of enzymes in many cases, and secondary inactivation of enzymes due to the presence of heat, organic solvents and the like is unavoidable upon the immobilization treatment.

It has been proposed to use molded products of chitin, which is an abundantly obtainable natural resource, to carriers for enzyme immobilization [JP-B-56-35, JP-B-53-10150 and JP-B-61-111686 (the term "JP-B" as used herein means an "examined published Japanese patent application")].

Several processes have been disclosed for the preparation of chitin moldings which comprises treating exoskeletons of crustaceans and insects with hydrochloric acid and sodium hydroxide to isolate chitin and then molding it in JP-A-61-53339, JP-A-61-64256, JP-A-61-129005 and JP-A-61-212302 (the term "JP-A" as used herein means an "unexamined published Japanese patent application").

Chitin obtained from exoskeletons of crustaceans and insects has α-type structure (hereinafter referred to as "α-chitin") which shows poor dope forming ability when treated by usually used means. Therefore, carriers prepared from this type of chitin without using a crosslinking agent are insufficient in strength and the like properties, and it cannot be made into proper thin membrane by the known methods. α-Chitin, therefore, has not yet been put into practical use as carriers for enzyme immobilization.

The present inventors have examined various chitin materials and found that β-type chitin (hereinafter referred to as "β-chitin") obtained from cuttlebones, especially β-chitin obtained from gladii of a cuttlefish belonging to the order Teuthoidea of the Mollusca, has excellent properties as carriers, can be made easily into enzyme immobilized carriers retaining high enzymatic activity and can be used in enzyme electrodes because of its moldability into thin membrane.

As described above, an enzyme sensor is constructed by equipping an electrode with an enzyme-immobilized membrane. With regard to the equipping techniques, however, there are many problems to be solved.

### SUMMARY OF THE INVENTION

An object of the present invention is to provide an enzyme-immobilized membrane formed into a thin film having proper substrate permeability, namely a substrate sensitive membrane, especially to be used as an electrode for use in multi-functional enzyme sensors, which improves in uniformity, miniaturization, safety and the like.

The present invention provides a substrate sensitive membrane having a thin metal film layer.

The present invention also provides a substrate sensitive membrane to be used as an electrode for use in multi-functional enzyme sensors comprising a membrane having no substrate sensitivity on one side of which two or more membranes each having a different enzyme immobilized therein are layered separately or overlayed and on another side of which a thin metal film layer is directly layered.

### BRIEF DESCRIPTION OF THE DRAWINGS

Fig. 1 is a response curve showing substrate responses when the substrate sensitive membrane of the present invention is used as an enzyme electrode. In Fig. 1, 1 shows a glucose response curve, 2 shows a hydrogen peroxide response curve and 3 shows the time of sample application.

Fig. 2 schematically shows the production process of the substrate sensitive membrane having a thin metal film layer according to the present invention. In Fig. 2, 4 is a β-chitin layer containing no enzymes, 5 is a first mask, 6 is an enzyme-containing chitin membrane, 7 is a second mask, 8 is deposited gold and 9 is a substrate sensitive membrane.

Fig. 3 shows an example of the enzyme electrode of the present invention. In Fig. 3, 10 is a gold-deposited site and 11 is an enzyme-immobilized site.

Fig. 4 is a calibration curve showing the enzyme-substrate response obtained in Example 3.

### DETAILED DESCRIPTION OF THE INVENTION

The membrane used for the substrate sensitive membrane of the present invention is preferably made from chitin, more preferably β-chitin.

Chitin, namely poly-β1,4-N-acetylglucosamine, is a principal structural polysaccharide found in arthropods, mollusks, ectoprocts and fungi, which resembles cellulose in various points, such as its white amorphous powder or fibrous form and its insoluble nature in acid, alkali and many solvents. It is insoluble also in cuprammonium solution. Since chitin has so good biocompatibility as to be decomposed *in vivo*, its application to surgical suture, wound coating and the like has been noted.

β-Chitin may be isolated, for instance, from cuttlebones. The substrate sensitive β-chitin membrane of the invention is described below.

Isolation of β-chitin from cuttlebones may be effected by treating the bones with sodium hydroxide and hydrochloric acid to remove protein and ash contents. In the case of using cuttlefish gladii as the source material, gladius is treated with about 1 N sodium hydroxide solution at 90°C for about 1 hour and then with about 0.1 N hydrochloric acid at room temperature for about 1 hour, followed by drying to obtain β-chitin.

An enzyme-immobilized carrier can be prepared in the following manner. β-chitin is thoroughly ground to a particle size of preferably 16 mesh or less and suspended in water to a concentration of from 0.1 to 20% by weight. The resulting suspension is stirred vigorously or subjected to freeze-thawing treatment. Within a short time, β-chitin absorbs water and the suspension increases in viscosity to finally become a dispersion. Thereafter, a predetermined amount of an enzyme solution is added thereto to make the mixture uniform. The resulting enzyme-containing β-chitin dispersion is dried and molded to obtain an enzyme-immobilized carrier.

Chitin and the enzyme-immobilized carrier can be made into membranes by molding techniques conventionally used, for example, the water dropping type papermaking. Namely, the enzyme-containing β-chitin dispersion obtained above may be poured and spread onto a papermaking filter, water is removed from under the filter and the residue on the filter is dried.

Any enzyme can be used to be immobilized to β-chitin. Examples of the enzyme include glucose oxidase, alcohol oxidase, invertase, pyruvate oxidase and the like. A plurality of enzymes may also be immobilized in combination, for example, glucose oxidase and peroxidase, glucose oxidase and catalase, glucose oxidase and glucoamylase, L-arginine decarboxylase and amine oxidase and the like combination.

According to the prior art technique, a membranous enzyme-immobilized carrier obtained in such a manner, namely a substrate sensitive membrane, is used for an enzyme electrode in combination with an electrode which detects reaction of the immobilized enzyme. According to the present invention, on the contrary, a thin metal film layer is formed directly on the substrate sensitive membrane to serve as an enzyme electrode. For example, a thin gold film layer is formed by means of evaporation on an enzyme-immobilized membrane carrier obtained by immobilizing an enzyme to β-chitin. Though not particularly limited, formation of the thin metal film layer may be formed by not only vacuum evaporation but also sputtering or electroless plating. In the case of the vacuum evaporation, a thin metal film layer is formed directly on the substrate sensitive membrane by usually used means.

A thin metal film layer may be directly formed on a β-chitin membrane having a plurality of enzymes immobilized thereon. It is preferable to prepare the substrate sensitive membrane by laminating two or more substrate sensitive membrane pieces each having a different enzyme immobilized therein on one side of a membrane having no substrate sensitivity. In this case, a thin metal film layer pieces are directly deposited on the other side of the membrane having no substrate sensitivity only at positions corresponding to the substrate sensitive membrane pieces, with masking unnecessary areas on the membrane.

The substrate sensitive membrane and the membrane having no substrate sensitivity preferably have a thickness ranging from 0.05 to 1.0 mm, while the thin metal film layer has a thickness ranging from 500 to 3,000 angstroms.

Gold is preferably used as a metal source of the thin metal film layer, taking safety and biocompatibility into consideration, but any other metal, such as platinum or silver, may be employed depending on the type of the enzyme electrode.

Thus, a substrate sensitive β-chitin membrane having a thin metal film layer is obtained, which can be used as an enzyme electrode.

Enzyme sensor techniques have been developed mainly in the field of clinical tests and are now applied to other field of analyses, such as food analysis, process analysis in fermentation industry and the like. Especially promising among such techniques is the enzyme electrode-aided analysis, which has already been applied to the analysis of the degree of fish meat freshness, contents of nucleic acid-related compounds and the like, because it has an advantage in that samples can be analyzed quickly, simply and easily in comparison with the known chemical analyses. Thus, the present invention provides an enzyme electrode which can be used even in such new analytical fields.

The following examples are provided to further illustrates the present invention, but are not to be construed to limit the scope of the invention.

### EXAMPLE 1

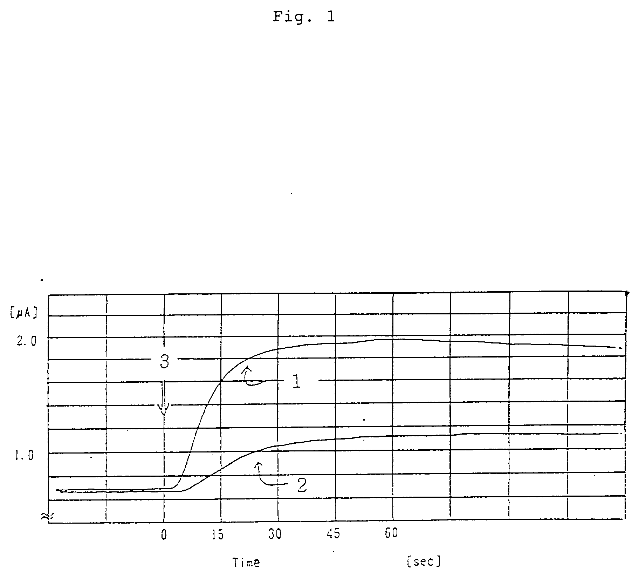

(1) A 1 kg portion of gladii of sagittated calamary was ground using a feather mill (5 m/m, screen bath), and the thus ground gladii was heated in 1 N NaOH solution at 90°C for 1 hour, washed with water and then soaked in 0.1 N HCl solution at room temperature for 1 hour. After washing with water, the resulting product was heated in 1 N NaOH solution at 90°C for 1 hour, washed with water and then dried for 5 hours in a 50°C oven to obtain 100 g of β-chitin.
(2) The thus obtained β-chitin was ground and mixed with water to give a final concentration of the dry ground chitin of 0.2% by weight, and the mixture was stirred vigorously to increase its viscosity and to give a dispersion. A 25 ml portion of the thus obtained dispersion was mixed with 25 ml of β-D-glucose oxidase (Sigma Chemical Co.) aqueous solution (2 mg/ml), and the mixture was molded by a water dropping type papermaking in a conventional manner. The resulting membrane was air-dried at 4°C for 15 hours to obtain a β-D-glucose oxidase-immobilized membrane. The amount of the enzyme was measured in terms of absorbance at 280 nm before and after the papermaking. As a result, it was found that 86% of the enzyme molecules were immobilized to the membrane.
(3) The β-D-glucose oxidase-immobilized membrane thus prepared was set in a vacuum evaporation apparatus, and gold was deposited onto one side of the membrane to a thickness of 2,000 angstroms. A wire was fixed on the thus deposited gold with silver paste, and the entire surface was coated with an epoxy resin to obtain an enzyme electrode. The resulting enzyme electrode was put into 10 ml of 50 mM phosphate buffer (pH 7.4) maintained at 37°C and, using a silver/silver chloride electrode as its counter electrode, a voltage of 0.6 V was applied. With stirring with a magnetic stirrer, to the resulting buffer was added hydrogen peroxide to a final concentration of 60 ppm or glucose to a final concentration of 4 mM. The results of the response are shown in Fig. 1.

### EXAMPLE 2

The gold-deposited membrane prepared in Example 1 was put into 0.1 M phosphate buffer containing 5 mg/ml of egg white lysozyme. When maintained at room temperature, the membrane was decomposed gradually.

### EXAMPLE 3

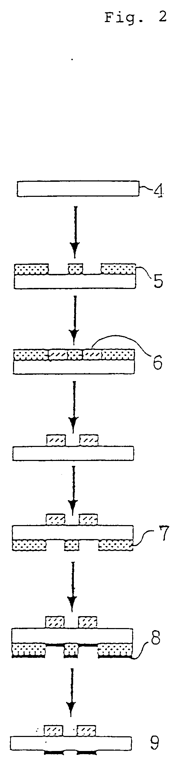

In order to integrate a plurality of sensors on a chip, an enzyme electrode was prepared in accordance with the procedure shown in Fig. 2. That is, one side of an enzyme-free β-chitin membrane (4) was covered with a plastic thin plate which is partially cut out as a mask (5), pieces of an enzyme-containing chitin membrane (6) were layered and then the mask was removed. The other side of the thus obtained substrate sensitive β-chitin membrane was covered with a mask (7) and coated directly with thin gold film pieces (8) only at positions corresponding to the substrate sensitive membrane pieces by means of vacuum evaporation. Thereafter, the mask was removed to obtain an enzyme electrode (9).

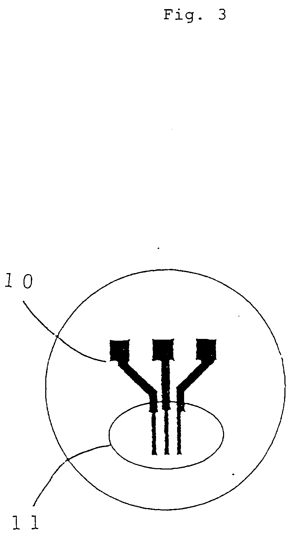

The mask to be used for the preparation of the layers of thin metal film and immobilized enzyme membrane may be in any optional shape, with an example of the mask shown in Fig. 3. Using the mask shown in the figure, an enzyme electrode was obtained in which pieces of thin gold film were formed [a metal-deposited site (10)] on one side of a chitin membrane, and pieces of an enzyme-immobilized β-chitin membrane layer were laminated [an enzyme-immobilized site (11)] on the backside at corresponding positions.

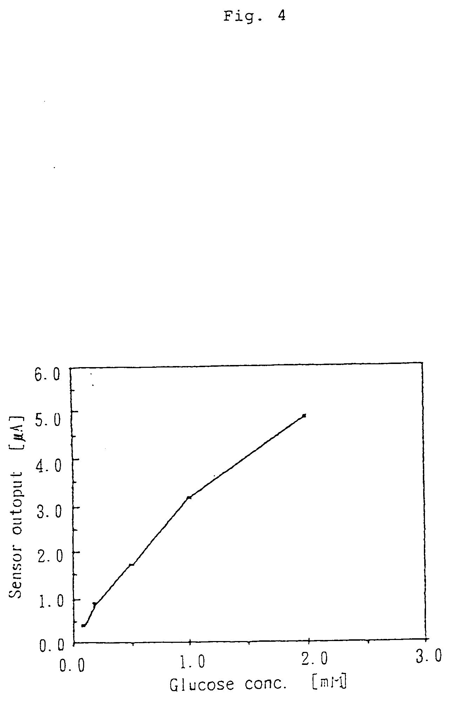

When a glucose oxidase-immobilized enzyme electrode was prepared in accordance with the procedure shown in Fig. 2 and its response to glucose was measured in the same manner as described in Example 1, a calibration curve shown in Fig. 4 was obtained.

Thus, it is apparent that the substrate sensitive β-chitin membrane having a thin metal film layer is markedly useful as an electrode for use in enzyme sensors, because its enzymatic activity is highly stable, it has biocompatibility as a whole, it can be made into a thin and small form and it can be handled easily and simply.

While the invention has been described in detail and with reference to specific examples thereof, it will be apparent to one skilled in the art that various changes and modifications can be made therein without departing from the spirit and scope thereof.

## Claims

1. A substrate sensitive membrane having a thin metal film layer.

2. The substrate sensitive membrane according to claim 1, which comprises a membrane having no substrate sensitivity on one side of which a substrate sensitive membrane piece is layered, and on the other side of which a thin metal film layer are formed at positions corresponding to the substrate sensitive membrane piece.

3. The substrate sensitive membrane according to claim 1, which comprises a membrane having no substrate sensitivity on one side of which two or more substrate sensitive membrane pieces are layered separately or overlayed, and on the other side of which a thin metal film layer are formed at positions corresponding to the substrate sensitive membrane pieces.

4. The substrate sensitive membrane according to claim 1, wherein said membrane is made from chitin.

5. The substrate sensitive membrane according to claim 1, wherein said membrane is made from β-chitin.

6. The substrate sensitive membrane according to claim 1, wherein said metal is gold.

7. An enzyme electrode comprising a substrate sensitive membrane having a thin metal film layer.

8. The enzyme electrode according to claim 7, wherein said substrate sensitive membrane comprises a membrane having no substrate sensitivity on one side of which a substrate sensitive membrane piece is layered, and on the other side of which a thin metal film layer are formed at positions corresponding to the substrate sensitive membrane piece.

9. The enzyme electrode according to claim 7, wherein said substrate sensitive membrane comprises a membrane having no substrate sensitivity on one side of which two or more of substrate sensitive membrane pieces are layered separately or overlayed, and on the other side of which a thin metal film layer are formed at positions corresponding to the substrate sensitive membrane pieces.

10. The enzyme electrode according to claim 7, wherein said membrane is a chitin membrane.

11. The enzyme electrode according to claim 7, wherein said membrane is a β-chitin membrane.

12. The enzyme electrode according to claim 7, wherein said metal is gold.
